# EUROPEAN PATENT SPECIFICATION

(11) **EP 2 366 329 B1**
(45) Date of publication and mention of the grant of the patent: **24.12.2014**
(21) Application number: 10157120.6
(22) Date of filing: 19.03.2010
(51) Int. Cl.: A61B 5/04

(54) **Nerve monitoring device**
Nervenüberwachungsvorrichtung
Dispositif de surveillance nerveuse

(43) Date of publication of application: 21.09.2011
(73) Proprietor: The Magstim Company Limited, Carmarthenshire SA34 0HR (GB)
(72) Inventor: Kartush, Jack, Farmington Hills MI 48334 (US)
(74) Representative: Baker, Thomas Edward

(56) References cited:
- US-A- 5 016 647
- US-A- 5 343 860
- US-A1- 2010 063 376
- US-B1- 6 266 548

## Description

### BACKGROUND OF THE INVENTION

The present invention relates to nerve monitoring, and more particularly, to a device to facilitate nerve monitoring.

A risk presented by thyroid surgery, parathyroid surgery, skull base surgery, or any other surgery in the space around the oropharynx, larynx, trachea or esophagus, is damage to the Recurrent Laryngeal Nerves ("RLN"). RLNs control the vocal cords, and damage to them can result in full or partial vocal cord paralysis. An issue with RLNs is that they are small and difficult to identify, particularly where surrounding tissue is bloodied, inflamed or otherwise disrupted due to surgery or trauma. Another issue is that simply trying to identify RLNs by touch can stretch or tear those nerves, which can result in hoarseness, difficulty in speech, aspiration of food or liquids (which can result in pneumonia), and life-threatening airway obstruction.

Accordingly, there have been recent efforts to use intraoperative RLN monitoring techniques, with the objective of reducing the risk of damage to the RLNs and subsequent vocal cord impairment or paralysis. One advocated form of RLN monitoring implements electromyography (EMG) to protect the nerves.

A common procedure in which laryngeal EMG is used is a thyroid surgery. In this procedure, a specialized endotracheal tube (ET tube) is placed through the patient's nose or mouth and into the trachea to assist in respiratory ventilation and/or to provide anesthesia. The ET tube also passes between the sets of laryngeal muscles, and typically rests adjacent the left and right posterior cricoarytenoid muscles. The specialized ET tube includes a pair of exposed, cylindrical wires on its external surface or embedded therein. These wires form electrodes that are intended to contact the various vocal muscles when the ET tube is (a) properly inserted at the correct depth, and (b) properly rotationally oriented relative to the trachea and larynx. These electrodes of the ET tube are capable of detecting EMG signals generated by an electrical probe. An example of such a specialized tube is disclosed in U.S. Patent 5,125,406 to Goldstone, which is hereby incorporated by reference.

During the procedure, a surgeon applies the electrical probe to the area in which he believes the RLN is located. If the electrical probe applies voltage to or near the RLN, the electrical pulse is carried to the vocal muscles (primarily the "thyroarytenoid muscles" along the vocal cords anteriorly and the "posterior cricoarytenoid muscles" posteriorly) through the RLN, which in turn causes contraction of the vocal muscles which generate their own electric pulse. The respective wire electrode on the ET tube facing the stimulated vocal muscles subsequently detects the electromyographic (EMG) response. The detecting electrode transfers a signal to a receiver or EMG monitor, which emits an audio or visual alarm. This output alerts the surgeon that the probe is close to the RLN so that the surgeon can confirm the nerve's location and minimize trauma in the probed location.

Documents US 5,343,860 and US 2010/0063376 A1 disclose systems according to the preamble of claim 1.

One commercially available instrument suitable for the above procedure is the Kartush Stimulating Dissection Instruments (KSD), which allow ongoing electrical mapping of the nerve's location during surgical dissection by simultaneous stimulation and surgical dissection. Education, however, is required of thyroid and other surgeons using the above procedure to assure appropriate Stimulating Dissection to minimize false positive and false negative stimulation errors.

Another challenge concerning the above procedure concerns minimizing false negative and positive recording errors, especially related to contact between the electrodes on the ET tube and the laryngeal muscles to monitor the RLN. It is frequently difficult to ensure adequate Electrode-Vocal Cord (EVC) contact both as the ET tube is being inserted in the patient and after the ET tube is positioned. In other words, the ET tube electrodes used to monitor the RLN can be difficult to accurately place, as well as difficult to maintain in proper position.

Obtaining sufficient EVC contact is limited by several factors. First, direct visualization of the EVC juxtaposition typically occurs only during intubation. Even if the ET tube is checked immediately after positioning in the patient, loss of appropriate EVC contact may go undetected if it is not repeatedly checked. Further, the anterior location of the larynx or a large, floppy epiglottis can prevent direct visualization of EVC contact, even with a laiyngoscope. Although this can be overcome by a flexible scope, the time and expense to add intermittent or ongoing flexible fiber optic endoscopy following standard intubation with a rigid laiyngoscope can make this procedure impractical.

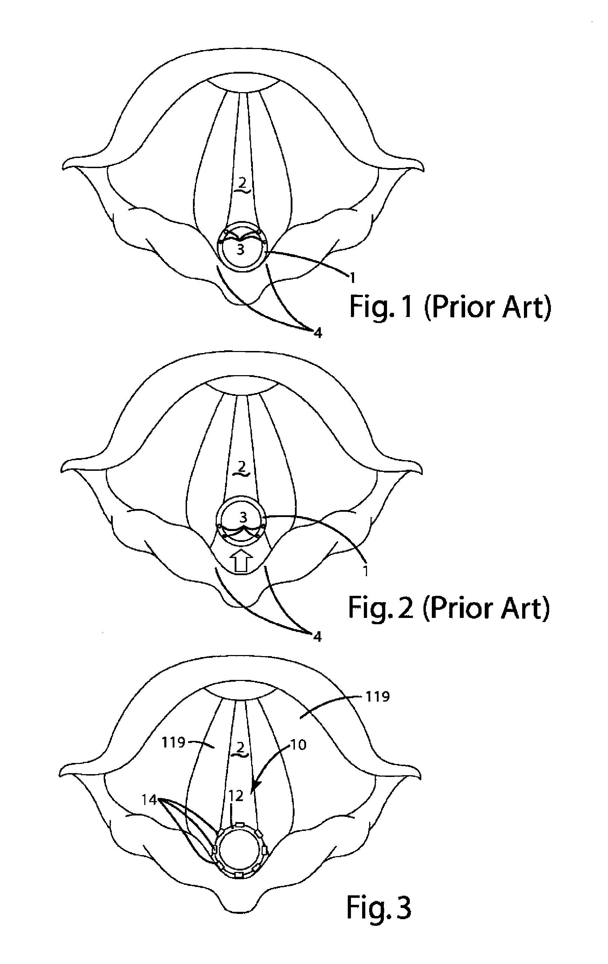

Second, the electrodes of the current and previous devices are positioned on a round ET tube - however, the aperture of the human glottis, i.e. the glottic opening, is triangular. This creates a fundamental mismatch between the geometry of the ET tube and the laryngeal surface, such as the glottic opening and other surrounding laryngeal muscles. An example of a conventional ET tube 1, including conventional wire electrodes 3, is shown in Fig. 1. As can be seen there, the ET tube 1 is circular, while the glottic opening 2 is generally triangular, which results in a mismatch between the ET tube and the laryngeal anatomical geometries, and subsequently contact with the target laryngeal muscles. There have been attempts to improve electrode contact by simply increasing the outer diameter of standard ET tubes to press the electrode on the target laryngeal muscles, which may be the vocal cords. These attempts, however, can lead to difficult and traumatic intubations, as well as the possibility of pressure-induced vocal cord injury, particularly during prolonged operations such as removal of skull base tumors.

Third, there can be anatomic variances in the pharynx and larynx that can force the ET tube to enter the glottis at an angle that reduces contact at the EVC interface, that is, the ET tube may be placed too anterior or too posterior to the laryngeal muscles. An example of the ET tube 1 being placed too anterior (see arrow) to the posterior criciaiytenoid muscles 4 so that the electrodes 3 do not have adequate contact with these target muscles 4 is illustrated in Fig. 2. Further, The ET tube may be inserted too deeply or too shallow, which can result in the electrodes being placed inferior or superior to the laryngeal muscles.

Fourth, inadvertent rotation of the ET tube about its longitudinal axis can skew the electrodes away from the target laryngeal muscles and minimize or eliminate proper contact. For example, as shown in Fig. 1, the electrodes 3 have been inadvertently placed opposite the target laryngeal muscles 4, thereby eliminating contact with those target muscles 4. Without contact between the electrodes and the vocal cords, the device may provide a "false negative error"-that is, the device might not emit an alarm indicating detection of the electrical impulse in the muscle. Thus, the surgeon may not appreciate the proximity of the RLN to the electrical probe. Rotation issues may also be exacerbated by a recent shift toward the use of a more rigid, reinforced ET tube (intended to make intubation easier). With this construction, minor rotation of the ET tube at the mouth can result in rotation at the vocal cords or generally within the laryngeal space.

Fifth, to compensate for inaccurate ET tube insertion depth, some ET tubes have increased the uninsulated contact area of the electrodes. This modification, however, can increase the possibility of a "false positive errors." For example, increased exposure of the tube's electrodes can detect inferior constrictor muscle activity. This inadvertently detected stimulation of the inferior constrictor muscle may be misinterpreted as vocal cord stimulation and proximity to the RLN by the electrical probe. Such false positive errors can lead to considerable anatomic disorientation of the surgeon.

Sixth, the EVC contact interface can dry over prolonged periods of contact. This drying can increase impedance which can reduce the detection of the EMG response. In a similar manner, too much moisture from secretions or intentionally applied lubricating jelly may cause shunting of the electrical response away from the electrodes, thereby reducing EVC contact.

Seventh, both false positive and false negative errors can be caused by improperly set coding parameters between the electrodes and the alarm monitor. For example, if the stimulus filter (Ignore Period) is set too long by a surgeon, it may filter out both the true response as well as the stimulus artifact.

Accordingly, there remains room for improving nerve monitoring devices to ensure that the monitored nerves are not damaged or impaired due to inadvertent contact or severing.

### SUMMARY OF THE INVENTION

A nerve monitoring device is provided to efficiently monitor a variety of nerves within a subject's body.

In one embodiment, the device can include a cannula and a sensor for monitoring a nerve. The device can be inserted into a body space at a desired depth of insertion and at a desired rotational orientation to monitor the activity of the nerve and/or an associated muscle(s).

In another embodiment, the sensor can be in communication with a processor to which the sensor outputs signals or data concerning electrical stimulation of the nerve and/or associated muscle caused by an electrical probe in electrical communication with the sensor. The processor can analyze the output of the sensor and can provide information to a health care provider, for example, a surgeon or nurse, concerning the nerve activity. This information can be indicative of the location of the nerve relative to the electrical probe, and can be output in the form of visual and/or audible output to the health care provider.

In yet another embodiment, the sensor can include structural elements that enhance contact between the sensor with the anatomic features, such as muscles, nerves or tissue, within a body space in which the cannula is inserted in an atraumatic manner. Optionally, the sensor can be of a geometric configuration that moves to conform to the geometric configuration of the body space within which the cannula is placed so that the sensors satisfactorily contact the target muscle and/or nerve.

In still yet another embodiment, where the device includes the cannula and sensor to enhance contact between the sensor and the anatomical featured, the sensor can include electrodes that are moveable, flexible, compressible and/or expandable. For example, the electrodes can be constructed from a soft, felt-like material, or some other flexible or expandable or compressible material or elements. With such a sensor, even where the cannula is geometrically dissimilar to the body space within which it is placed, the electrodes joined with the cannula can overcome this mismatch, and satisfactorily contact the target muscle(s) and/or nerve to monitor the nerve.

In even another embodiment, where the device includes the cannula and sensor to enhance contact between the sensor and the anatomical features, the sensor can be in the form of a multi-electrode array, having multiple electrodes positioned around the cannula in a predetermined configuration. This array of electrodes can compensate for any rotational error of the cannula within the body space relative to the target muscle(s)/nerve. This embodiment goes beyond standard monopolar or bipolar electrodes by allowing complete user selection of whichever electrode combination provides clinically the most useful montage.

In another, further embodiment, where the device includes the cannula and sensor to enhance contact between the sensor and the anatomical features, the device or cannula can further include a support element which, when placed in the body space, expands to substantially fill at least a cross section of the body space. The electrodes can be joined with the surface of the support element and configured so that they move and/or reorient relative to the body space. Where the support element expands sufficiently so that the surface engages a target muscle(s) or nerve within the body space, the sensor likewise can contact the muscle(s) and/or nerve to monitor the nerve. An optional example of such an embodiment can include an support element constructed from a material having sponge-like properties, that is, it expands when wetted. The sensor can include electrodes connected to sensory elements, such as caps, located on or adjacent the surface of the support element. The sensor elements can move from a position proximal the cannula, to a position distal from the cannula, and adjacent a target nerve and/or muscle(s), when the expanding element is activated, for example, when it is wetted.

In a further embodiment, where the device includes the cannula and sensor to enhance contact between the sensor and the anatomical features, the cannula can be constructed so that its external geometry is conformable to the body space within which it and the sensor is placed. For example, the cannula can be constructed to include, or joined with a support element constructed from, a material that selectively and atraumatically expands or compresses or otherwise changes in shape, or moves an exterior surface of the cannula. In turn, the exterior surface of the cannula generally conforms to the anatomic geometry of a body space with which the cannula is positioned. In its altered configuration, the cannula or support element constructed from the above material can urge and/or maintain the sensor, which is attached adjacent the conforming material, into contact with the target nerve/muscle(s) to ensure appropriate monitoring.

In still a further embodiment, where the device includes the cannula and sensor to enhance contact between the sensor and the anatomical features, the cannula can include a cannula wall of a thickness sufficient to enable the wall to flex and/or deform when positioned in a body space adjacent a target muscle and/or nerve. Optionally, the cannula wall can be constructed of a compliant, flexible material that reactively alters the geometric cross section of the cannula when the cannula is placed in a body space adjacent a target muscle/nerve. As an example, the cannula can include walls constructed from a polymeric material and of a thickness that enables the wall(s) to flex or deform under forces encountered when a cannula is inserted in an internal body space. Optionally, the cannula can be an ET tube, adapted for insertion into a laryngeal space. The wall(s) of the ET tube, when positioned through the generally triangular laryngeal space, can flex and change shape so that the wall(s) become generally triangular, conforming to the triangular laryngeal space, such as the glottis. A sensor joined with a surface of the cannula can be urged into contact with the muscles and/or nerves in laryngeal space. The generally automatically conforming cannula can enhance the contact of the sensor, for example, an electrode, with the target muscle(s) and/or nerve, for example, one or more laryngeal muscles, to properly monitor the nerve(s).

In still yet a further embodiment, the device can include a cannula, an optional sensor for monitoring a target nerve/muscle(s) and an alignment element. The cannula can be any surgical cannula, for example, an ET tube. The sensor can be an electrode or other sensor that is capable of sensing nerve or muscle activity. The alignment element can be configured and can include an indicator element that assists in ensuring that after insertion of the sensor into an internal body space of a patient, the sensor is aligned with the target nerve or muscle. The indicator element can output signals or information externally, through body tissue, for example, transcutaneously, to a health care provider. The signals optionally can convey information regarding the insertion depth of the ET tube, as well as rotational alignment of featmes of the ET tube and/or sensors relative to a target nerve/muscle(s). The indicator element may act as either transmitter or receiver.

In an even further embodiment, the device including the cannula, the sensor and the alignment element can be configured with the alignment element joined in a fixed relationship to the cannula. The alignment element can include at least one alignment indicator that provides visual, aural or other signaling output to a health care provider to convey information concerning the rotational orientation of the cannula relative to the space and/or the depth of the cannula into the body space. Optionally, the alignment indicator can include elements that light in a manner that is visible exteriorly to the body in which the device is placed. Further optionally, the alignment indicator can be or include a transmitter and/or receiver that communicates with a corresponding device placed externally in relation to the body space.

In still another, further embodiment, the device including the cannula, the optional sensor and the alignment element can include multiple alignment indicators corresponding to different portions of the cannula. Optionally, the sensor can include one or more electrodes configured and oriented in a predetermined spatial relationship relative to the cannula and/or the alignment element. The electrodes can be configured to contact and measure the response (if any) of a muscle/nerve within the body space where a nerve associated with the muscle is electrically stimulated, for example, by a stimulating probe. In one exemplary context, where an ET tube includes a cuff and an insertion tip, the alignment indicator can include a first alignment indicator joined with the insertion tip of the ET tube, another adjacent and below the cuff, and another adjacent but above the cuff. The indicators can illuminate or otherwise provide output through the tissue of the neck that a health care provider can visually or otherwise perceive and assess the location of these indicators, and thus the different parts of the ET tube, in the laryngeal space. If the health care provider perceives that the alignment indicators are out of their proper location, for example, the ET tube tip indicator is not far enough in the trachea, or an alignment indicator is rotated relative to a preferred location, the health care provider can take corrective action and reorient the ET tube to an appropriate orientation and/or position within the laryngeal space.

The device described herein provides a simple and efficient construction for atraumatically positioning and optionally maintaining a cannula within unique anatomical geometries of an internal body space. The device can provide reliable contact between sensors associated with the cannula and target muscles/nerves. Accordingly, the associated nerve and its location can be readily and reliably ascertained by a health care provider. This can prevent undesirable damage to or impairment of the nerve, particularly during surgery in a location near the nerve. Further, where an alignment element is included, the device can enhance measurement of stimulated muscles/nerves by generally enhancing sensor placement and/or cannula placement. Where an alignment element is associated with the cannula, that element can enhance proper placement and rotational orientation of the cannula within the respective body space.

These and other objects, advantages and features of the invention will be more fully understood and appreciated by reference to the description of the current embodiment and the drawings.

### BRIEF DESCRIPTION OF THE DRAWINGS

Fig. 1 is a top view of a prior art endotracheal tube including electrodes that are improperly positioned within the glottic opening, and out of contact with posterior target muscles;
Fig. 2 is a top view of the prior art endotracheal tube including electrodes that are improperly positioned within the glottic opening, and out of contact with anterior target muscles;
Fig. 3 is a top view of a device of a current embodiment in the form of an endotracheal tube including a multisensor array positioned within the laryngeal space;
Fig. 4 is a side sectional view of the device of the current embodiment positioned within the laryngeal space;
Fig. 5 is a side view of a first alternative embodiment of the device including one type of sensors;
Fig. 6 is a perspective view of a portion of the device of the current embodiment illustrating one type of moveable electrodes;
Fig. 7 is an end view of a portion of the device of the current embodiment illustrating the moveable electrodes of Fig. 6 conforming to anatomical geometry;
Fig. 8 is a top view of the device including the moveable electrodes of Fig. 6 conforming to the anatomical geometry of the laryngeal space;
Fig. 9 is a perspective view of a second alternative embodiment of a portion of the device illustrating another type of moveable electrodes;
Fig. 10 is an end view of a portion of the device of the second alternative embodiment illustrating the moveable electrodes of Fig. 9 conforming to anatomical geometry;
Fig. 11 is a top view of the device including the moveable electrodes of Fig. 9 conforming to the anatomical geometry of the laryngeal space;
Fig. 12 is a perspective view of a third alternative embodiment of a portion of the device;
Fig. 13 is a section view of the device of the third alternative embodiment, taken along lines 13-13 of Fig. 12, conforming to anatomical geometry;
Fig. 14 is a top view of the device of the third alternative embodiment conforming to the anatomical geometry of the laryngeal space;
Fig. 15 is a perspective view of a fourth alternative embodiment of a portion of the cannula of the device including a support element;
Fig. 16 is an end view of the device with the cannula including the support element of the fourth alternative embodiment conforming to anatomical geometry;
Fig. 17 is a top view of the device with the cannula including the support element of the fourth alternative embodiment conforming to the anatomical geometry of the laryngeal space;
Fig. 18 is a perspective view of a fifth alternative embodiment of a portion of the cannula of the device including a support element;
Fig. 19 is an end view of the device with the cannula including the support element of the fifth alternative embodiment illustrating expansion and compression of the support element; and
Fig. 20 is a top view of the cannula including the support element of the fifth alternative embodiment conforming to the anatomical geometry of the laryngeal space.

### DESCRIPTION OF THE CURRENT EMBODIMENT

A current embodiment of the device for monitoring nerves to detect nerve and/or muscle activity is illustrated in Figs. 3-4 and 6 and generally designated 10. The device 10 can include a cannula 12, sensor 14 and an optional output element 40, as well as an optional electrical probe 50.

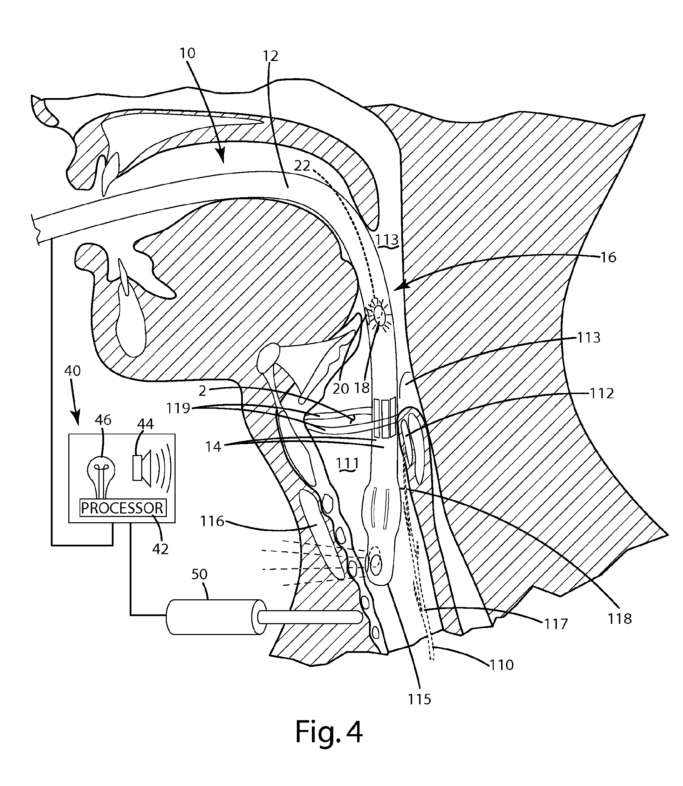

In general, the sensor 14 and probe 50 can be in communication with the output element 40. As shown in Fig. 4, a surgeon can engage the probe at a location where a target nerve, such as a recurrent laryngeal nerve 110, is suspected to be located. The probe 50 provides an electrical impulse, which in turn can be transmitted through the target nerve, to an associated target muscle, such as a laryngeal muscle, for example, posterior cricoarytenoid muscles 112 and/or the vocal cords 119. The subsequent activity of the target muscle can be measured or otherwise sensed by the sensors 14, and output to the output element 40 based on the measured response. The output element 40 can output information or an alert as to the location of the target nerve relative to the probe 50. In which case, the surgeon generally can perceive the location of the target nerve, and avoid further activity in the area so as to prevent unwanted damage or impairment to the target nerve.

While the embodiments herein are described in connection with a particular cannula, that is, an endotracheal tube used in the laryngeal space, it is to be understood that the device can be used in virtually any internal body space to monitor virtually any target nerve for purposes of avoiding unwanted damage or impairment to that nerve. For example, the device can be used in prostate, abdominal, pelvic or rectal surgery to prevent damage or impairment to associated nerves, e.g. pelvic nerves, pudendal nerves, etc. Alternatively, the device can be used to locate nerves that are to be rendered inoperative or to be used for acute or chronic neural stimulation.

As shown in Figs. 3-4 and 5-8, the device of the current embodiment can include a cannula 12, which can be any device known to those of skill in the art as being insertable into a patient. For example, the cannula can be in the form of an endotracheal tube (ET tube) used in procedure conducted in or around the laryngeal space 111 or oropharynx 113, as shown and described herein, or in the form of other tubular or exploratory devices used for other procedure, such as prostate surgery, rectal surgery, colon surgery, or other surgical or investigative procedure.

Where implemented as an endotracheal tube, the cannula 12 can be used in anesthesia, intensive care, neonatal care and emergency medicine for airway management and mechanical ventilation. In use, the cannula, or ET tube 12 can be inserted through a patient's laryngeal space 111 and into the trachea 117 to ensure that the patient's airway is open by providing alignment and position of the tube relative to the glottis and the carina.

An exemplary procedure in which the device 10 may be used to monitor a nerve is thyroid surgery, as noted above. In such a procedure, the ET tube is inserted as shown in Fig. 4 through the glottic opening 2, with the sensors generally are positioned in the laryngeal space 111. The target nerves for monitoring in such a procedure is the recurrent laryngeal nerves (RLN), which are generally adjacent the thyroid glands 116. The target muscles, with which the RLN are associated, can be the left and right thyroarytenoid and right and left posterior cricoarytenoid muscles (PCAs). These muscles will exhibit activity when the RLN are stimulated, for example, by a probe 50 exerting an electrical stimulus adjacent or directly on the RLN. Due to variability in electrical responses from patient to patient, the optional targeting of multiple laryngeal muscles including these posterior and anterior muscles can in some cases improve the opportunity to detect small responses. For example, in addition to the PCAs, it is helpful to monitor the thyroarytenoid muscles within the vocal cords 119. The device 10 can be outfitted with multiple sensors that align with the vocal cords 119 in addition to the sensors that align with the PCAs when the cannula is properly positioned. Depending on the desired monitoring activity, a health care provider can select which target laryngeal muscles are contacted by the respective sensors, and subsequently, what muscle activity is output from the output element 40.

Optionally, a variety laryngeal nerves can be monitored with the device 10, depending on the procedure. For example, in addition to the RLNs, laryngeal nerves such as the non-recurrent nerves, superior nerves, inferior nerves and/or the vagus nerves may be monitored. Of course, where the device is used in other body spaces, other nerves may be monitored. Likewise, a variety of other muscles may be targeted for such monitoring, depending on the internal body space within which the device is used.

Returning to the general description of the ET tube, there are many types of such tubes. For example, ET tubes range in size from 3 mm to 10.5 mm in internal diameter. Different sizes of tubes are chosen based on the patient's body size with the smaller sizes being used for pediatric and neonatal patients. ET tubes having internal diameters larger than 6 mm usually include an inflatable cuff (which is not shown in Fig. 4 for simplicity). The ET tubes can also be constructed from a variety of materials, such as polymers.

The cannula 12 can be constructed from a biocompatible material that is either disposable or sterilizable. The cannula 12 can be formed of a plastic and can include a coating on the exterior surface 13 if desired. For example, the coating can be used to enable easier insertion of the cannula 12, or can include a material that limits or prevents an adverse reaction in the patient after insertion of the cannula 12.

The device 10 of the current embodiment can include sensors 14. As used herein, sensors can be anything that is able to detect nerve activity. Examples of suitable sensors include sensors having electrodes that detect electrical or pulse stimulation by an electrical probe, as well as chemical sensors. Suitable chemical sensors can be sensors that detect an increased presence of a chemical or specific compound that is associated with a change or modulation in nerve activity. For example, calcium or potassium sensors can be used.

As illustrated in Fig. 3, 4 and 6, the sensors 14 can be in the form of a multi-sensor array that surrounds a portion, for example, a majority of an outer periphery of the cannula. In this array configuration, the larger number of sensors generally allow those sensors to conform to the anatomical geometry of the laryngeal space 111 so that at least one, two, or more of the sensors 14 are in monitoring proximity (defined below) to a target muscle, for example, a laryngeal muscle, such as the PCAs, thyroarytenoids, etc, and subsequently can measure activity of one or more of those muscles upon stimulation. Further, the array of sensors can be configured on a multiple channels. Thus, the array allows monitoring from different sensors (selected by a health care provider) and areas of the laryngeal space, around the cannula 12, and optionally, of different target laryngeal muscles (e.g., PCAs and/or vocal cords). This, in turn, can compensate for inadvertent cannula rotation within the laryngeal space as well as variations in muscle activity and monitoring in a variety of patients.

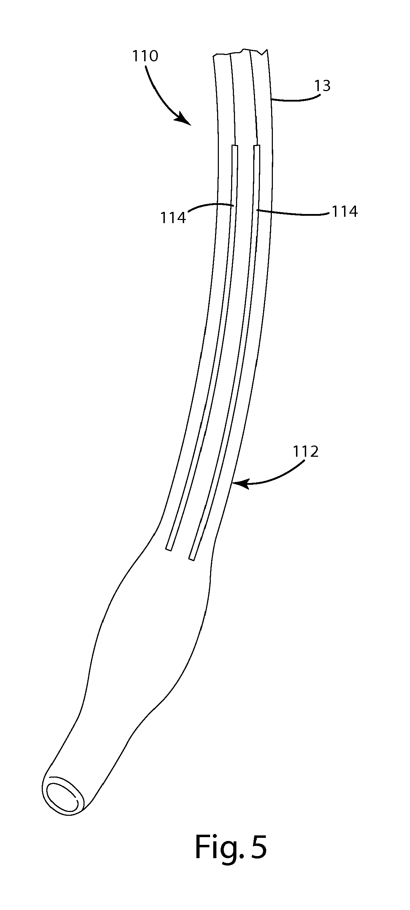

Optionally, the multi-sensor array 14 can include eight electrodes, with four on the left and four for the right sides of the cannula 12, in a generally symmetric orientation about the longitudinal axis of the cannula 12 as shown in Fig. 3. These electrodes can be connected to a nerve monitor with separate electrodes attached for ground and anode (stimulus return) on the sternum, for example. Further optionally, the electrodes in the array can be color coded, and can extend along the tube parallel to their laryngeal position until they branch off at the oral end of the cannula to help orient the health care provider to their deeper laryngeal position, as shown in the alternative construction of Fig. 5, which is described more below.

In general, a sensor, when in monitoring proximity to a target muscle or nerve, can measure activity of a muscle by simply detecting activity, or by detecting and measuring the level of activity against a predetermined value of activity. As used herein, monitoring proximity means that the sensor is close enough to the target muscle or nerve to detect that the muscle or nerve has been stimulated, or is undergoing some type of activity in response to a probe or other stimulation, e.g. mechanical manipulation. For example, monitoring proximity can mean that the sensor is close enough to the target muscle to detect electrical stimulation of the muscle. As another example, monitoring proximity can mean that a chemical sensor is close enough to the target muscle to detect a chemical change in the muscle indicative of stimulation or activity.

While shown in Figs. 3, 4 and 6 as including a multi-sensor array positioned sensors around a majority of the cannula 12, the sensors can be combined in a single sheet sensor subdivided into separate detection zones. These zones can be calibrated so that the sensor 14 or output element 40 can determine which zones are detecting muscle activity or stimulation, and subsequently can communicate that information to the output element 40 to assist a health care provider in determining the rotational orientation of the ET tube 12 within the laryngeal space 111.

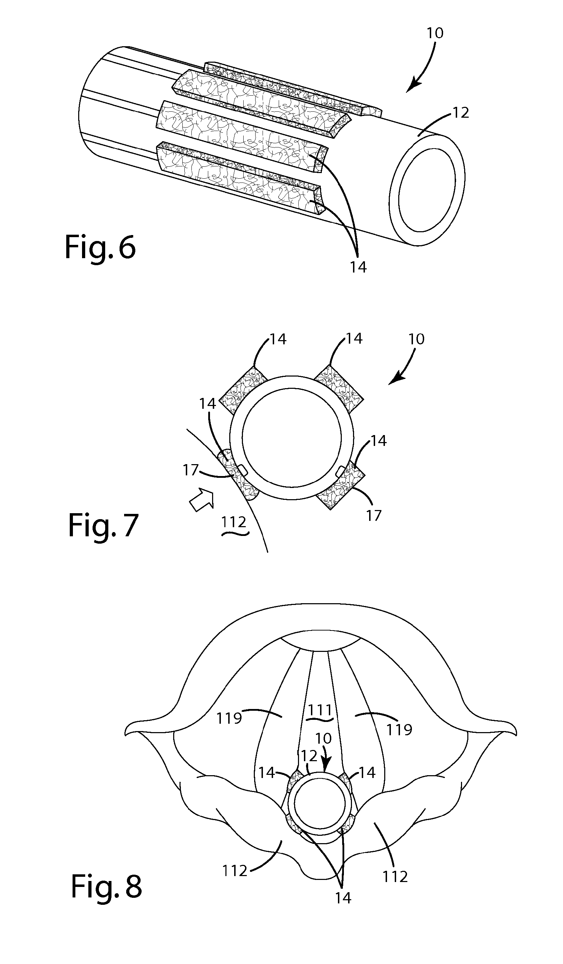

Further, while the embodiments in Figs. 3, 4 and 6 show eight sensors in an array that circumferentiate the cannula 12, fewer sensors can be used. For example, as shown in Figs. 7 and 8, the device may only include one, two, three, four or other numbers of sensors, configured and spaced on and around the ET tube so that when the ET tube is properly spaced, each of those sensors contact respective target laryngeal muscles. As mentioned above, due to the array configuration that surrounds a portion of the cannula 12, different sensors can be adapted to align with different laryngeal muscles, for example, certain sensors can align with the vocal cords 119, while others can align with other target laryngeal muscles 112, for example PCAs.

In some cases, the array configuration can assist in enhancing and/or maximizing sensor contact with target muscles both anteriorily and posteriorily. This can be helpful across a variety of patients. For example, in some patients there appears to be a maximal muscle response from the vocalis muscles in the vocal cords, that is, the thyroartenoid muscles, 119 (anteriorly), whereas in other patients, the response appears maximal at the PCAs 112. Where multi-channel recording of activity detected by different sensors is optionally implemented, this can further enable the health care provider to choose electrode pairs or groups that are suitable for every patient, despite anatomic differences. Further, where the optional multi-sensor array is used, responses may be detected both anteriorly and posteriorly to assure that any response was detected, that is, to avoid a false negative error where a true muscle contraction is not detected.

Optionally, the sensors can also extend along the ET tube for a greater distance. For example, as shown in the first alternative embodiment of the device 110 in Fig. 5, the sensors can be in the form of electrodes, 114 that extend along the tube for at least a or more of its length. The sensors are of a sufficient length that they are visible in the oropharynx or mouth to help orient the health care provider to the position of those electrodes in the laryngeal space. Further optionally, additional recording sensors can be placed on the tube in locations to engage convenient "non-relevant," that is, non-target, muscles to help distinguish artifact from true responses from target laryngeal muscles.

The sensors 14 used with the device 10 can vary in construction. As noted above, the sensors 14 can be in the form of exposed wire electrodes or plates that are in communication with the output element 40 and/or the probe 50. Generally, without some modification, these types of electrodes are fixed and immoveable relative to the cannula. As illustrated in Figs. 6-11, however, the sensors of some embodiments herein can be reconfigurable from a first configuration to a second different configuration where the sensor conforms to the anatomical geometry of the space within which it is placed. As an example, a reconfigurable sensor can include moveable electrodes. As used herein, a moveable electrode means an electrode that includes at least one portion that moves relative to another portion or to the cannula or to the structure to which the electrode is attached, in order to allow the portion or the entire electrode to conform to the anatomical geometry of the space within which it is placed. As another example, a reconfigurable sensor can be configured so that it changes in shape, size or orientation relative to the cannula to which it is attached, and in so doing, conforms better to the anatomical geometry of the space within which it is placed. Optionally, the reconfigurable sensors, and in particular, the moveable electrodes, can be self-reconfigming in that they are adapted to move, be moved, expand or otherwise reconfigure or alter in size to provide increased contact and/or maximized monitoring.

Referring to Figs. 3 and 6-8, the reconfigurable sensor can be in the form of a moveable electrodes having a plurality of fibers, strands or filaments 17 that are included in a pad like element, which resembles a felt or fibrous type structure. Any electrically conductive pad including fibers, strands or filaments suitable for sensing electrical activity in a target muscle is suitable for use with the device. Other materials that optionally may be used in the pad, or generally with the moveable electrodes, include materials already used in surgery (e.g., brain cottonoids) soft, expandable materials such as Merocel® (commercially available from Medtronic Xomed, Inc.), or other materials used for epistaxis and sinus surgery. In some applications, the material used to construct the sensor can expand when wetted with moisture (either before insertion, or by the patient's secretions), enhancing contact with the target muscles while reducing trauma to the tissue. In addition, the pad can retain moisture to minimize impedance.

In operation, the sensor 14 can compress when engaged with an external force, so that its thickness decreases as shown in Fig. 7 upon engagement of the tissue 113. With this compression, the sensor generally reconfigures in shape yet maintains engagement with the tissue. Fig. 8 illustrates the device 10 with moveable sensors positioned in the laryngeal space, adjacent the target muscles 112 (e.g., PCAs), and the vocal cords 119, for monitoring the RLN. There, the sensors have been pushed against the target muscles 112, 119 and have generally reconfigured so as to provide suitable contact between them and the muscles 112 and 119. Because they compress, the sensors 14 also can reduce the forces exerted on the target muscles 112, 119 and other laryngeal tissue. This, in turn, can reduce trauma to those muscles when the device is used.

The sensors 14 shown in Figs. 6-8 can be joined with the cannula 12 at a predetermined location that corresponds to the target muscle or nerve to be monitored upon insertion of the ET tube 12.

The sensors 14 can further include wires or other elements that are in communication with the output element 40. The sensors 14 can be joined directly to the exterior surface 13 of the cannula 12 via an adhesive, or can be embedded or molded within the cannula components, or can be joined to another structural element that is placed about the ET tube.

For example, the sensors 14 can be attached to an adjustable, removable sleeve (not shown) that can be used as a retrofit for currently available cannulas. For example, the sleeve can be manufactured separately and affixed to the cannula 12 before or after intubation. The latter allows a conventional ET tube of normal diameter to be positioned in the laryngeal space, followed by a sleeve slid over the ET tube. In this case, the ET tube can act as a stylet for the sleeve. Optionally, the sleeve can be adjusted up or down on the ET tube, and rotated about the ET tube.

The sleeve can include pockets (not shown) into which the sensors 14 are placed. Alternatively, the sleeve can include sensor holding strips that maintain the sensors 14 in place on the exterior surface of the sleeve. The sensors 14 can either be integrated within the material of the sleeve or can be added post production thereby enabling the sensors to both be removed and be changed depending on the type of sensor needed.

As noted above, the sensors 14 also can be attached directly to the exterior surface of the cannula 12. In such a configuration the sensor can be attached via surgical or other adherence technique that enables attachment of the sensor 14 without altering the functionality of the sensor 14. For example, if the sensor is a chemical or compound sensor, the adhesive can be selected so that it does not inhibit the function of the sensor. As stated previously, the sensors 14 can be formed of a compressible material that enables the cannula 12 to be inserted into the patient without causing undue trauma to the patient's airway, laryngeal space, or other internal body space.

The device 10 of the current embodiment can also include an output element 40, which can be in communication, either via a direct electrical wire or wirelessly, with the sensor. In general, the output element can be an external EMG monitoring device that provides output indicative of measured activity of the target muscle and/or the target nerve when the probe 50 is positioned adjacent or on the target nerve. As shown in Fig. 4, the output element can include a controller or processor 42 that receives signals or data from the sensors 14, process as the signals, and outputs information or an alarm to a health care provider as to the sensed muscle activity, and thus the proximity of the probe 50 to the target nerve. The output of the element 40 can be an audible alarm from a speaker 44, and/or a visual alarm via a light 46 or screen, or by movement, such as vibration of the probe.

The device 10 of the current embodiment can also include an optional alignment element 16 including one or more alignment indicators 18, 20, 22 which provide output to a health care provider as to the location of the sensors 14, or other components of the device, within the internal body space. The alignment element 16 can be any device 16 capable of providing to the user an indication of the position of the sensors which can assist in appropriate sensor location. This in turn, can increase the accuracy of the nerve monitoring, and thereby limit the risk of unwanted nerve impairment and/or damage. The alignment element 16 can provide ongoing feedback to the user either as a receiver or a transmitter. The feedback can be in the form of a sound/alarm, a visual indicator, a vibration, electromagnetic energy or other form that provides position status of the sensor 14.

The alignment element 16, and in particular, the alignment indicators 18, 20, 22 can be located in a variety of locations. Optionally, the alignment indicators 18, 20, 22 can be configured and oriented in a predetermined spatial relationship relative to features of the cannula 12 and/or the sensors 14. For example, the cannula 12 can include a cuff 118 and an insertion tip 115. The alignment element 16 can include a first set of alignment indicators joined with the insertion tip 115 of the cannula 12, and another set above the sensors 14 so that the alignment indicators are viewable in the oropharynx or mouth. The indicators 18, 20, 22 can illuminate or otherwise provide output through the tissue of the neck, or in the mouth, so that a health care provider can visually or otherwise perceive and assess the location of these indicators, and thus the different parts of the ET tube, in the laryngeal space. If the health care provider perceives that the alignment indicators are out of the appropriate location, for example, the ET tube tip indicators are not far enough in the trachea, or an alignment indicator is rotated relative to a preferred location, the health care provider can take corrective action and reorient the ET tube to an appropriate orientation and/or position within the laryngeal space.

As shown in Fig. 4, the alignment indicators can be in the form of light emitting diodes 18, 20, 22 or other electromagnetic transmitters. Optionally, the alignment element 16 can function by either transmitting to, or receiving from, external devices. Further optionally, insulated wires can connect the alignment indicators 18, 20, 22 to a power source (not shown) such as a disposable battery, a re-usable and/or rechargeable batters, a power some associated with the output element 40, or some other power some.

Again, in general, the alignment indicators 18, 20, 22 can provide readily understandable indications of whether the sensors 14 are properly aligned to provide accurate nerve monitoring. As an example, referring to Fig. 4, the indicators can be light emitting diodes 18, 20, 22 that are color coded to assist in determining ET tube position. The lights can be coded, for example with a red light 22 indicating a right side of the cannula, a blue light 18 indicating a left side of the cannula, and a yellow light 20 indicating a midline of the cannula 12. Generally, different emitted frequencies along the electromagnetic spectrum can differentiate between the individual transmitters, thus allowing accurate assessment of position or alignment in multiple planes. Alternatively, the alignment element 16 can implement transillumination, such as fiber optic illumination. With this type of illumination, fibers transmit light from an external some to illuminate the lateral and anterior borders of the cannula, thereby indicating the position of the sensors.

The embodiments herein and shown in the figures can enhance electrode-vocal cord contact, or generally can enhance sensor to target muscle contact, while optionally providing expedient feedback of position of the cannula within the respective body space. The different components of the embodiments may be used singly or in combination.

Use of the device 10 of the current embodiment will now be described in the context of monitoring an RLN in the laryngeal space. Of course the device can be used in the same or other internal body spaces with other muscles or tissue to monitor other nerves.

To begin, the cannula 12, complete with sensors 14, is inserted into the desired body space of the patient. In general, the sensor 14 can enable the health care provider to assess the location of the nerve to be monitored. While one purpose of monitoring the nerve can be to avoid damage or impairment of the nerve, another can be to detect the location of a nerve that is to be treated, and monitor the progress of a surgery or procedure designed to ablate, section, damage, reduce function or render useless the nerve. Another use would be to locate a nerve to allow stimulation, e.g. acute or chronic neural stimulation.

After insertion, and if included with the device, the optional alignment element 16 can be used by a health care provider used to ensure the sensors 14 are properly located adjacent the target muscle or nerve, and generally within monitoring proximity relative to the target muscle or nerve. For example, after insertion into the patient, transillumination of the alignment indicators 18, 20, 22 through the tissue of the subject near the sensor 14 (or other electromagnetic energy) allows assessment of ET tube 12 position transcutaneously, without the need for repeated endoscopy.

More specifically, immediately following intubation with a visual check of the ET tube 12 position, the alignment indicators 18, 20, 22 can be connected to the power source. Appropriate ET tube 12 position is determined by visualizing the transilluminated location of the LEDs 18,20,22 to assess correct depth and rotation of the ET tube 12. The optional alignment element 16 can be turned off, used intermittently or can remain powered to provide output to a health care provider regarding the location and rotational orientation to the ET tube 12, and to ensure it does not rotate or move during the surgery or procedure.

After insertion, the sensors 14 and an optional probe 50 can be connected to the output element 40, as well as a Stimulating Dissectors or other nerve stimulators. Further, the sensors 14 can be actuated to monitor nerve activity. When multichannel recording devices are available, additional sensors, or electrodes, optionally can be placed in monitoring proximity to non-relevant muscles to act as a control to rule out artifact and thereby reduce false positive nerve activity errors. In addition, impedances can be tested and a tap test performed on the larynx to further assess integrity of the set up. The initial stimulus intensity is typically set to 1 mA with alterations in the current based on clinical indications.

With the different elements appropriately connected, the health care provider can engage the probe 50 at a location where a target nerve, such as a recurrent laryngeal nerve 110 (Fig. 4), is suspected to be located. The probe 50 provides an electrical impulse, which in turn can be transmitted through the target nerve, to an associated target muscle, such as a laryngeal muscle, for example a posterior cricoarytenoid muscle 112 and/or a vocal cord 119 (Figs. 4, 8). The subsequent activity of the target muscle can be measured or otherwise sensed by the sensor 12, and output to the output element. The output element 40, based on the measured response, can alert the health care provider as to the general proximity of the target nerve relative to the probe.

Optionally, where the multi-sensor array 14 (Figs. 3-4, 5-8) is attached to an ET tube 12, that array can provide monitoring from different areas of the laryngeal space 111, thereby compensating for inadvertent ET tube rotation and allowing multiple recording modalities. The sensors can detect EMG responses from the laryngeal muscles arranged around the cannula, for example, the PCAs and the vocal cords. Further optionally, the multi-sensor array 14 can minimize the deleterious effects of the ET tube rotation by allowing the surgeon or technician flexibility in choosing the suitable recording montage for each patient. For example, the health care provider can choose to monitor all channels, and thus all target muscle electrical activity detected by all the sensors. Alternatively, the health care provider can monitor selected channels, corresponding to specific sensors in the multi-sensor array based on impedance testing and responses to electrical stimulation. Further alternatively, the health care provider can simply monitor in monopolar or bipolar modalities.

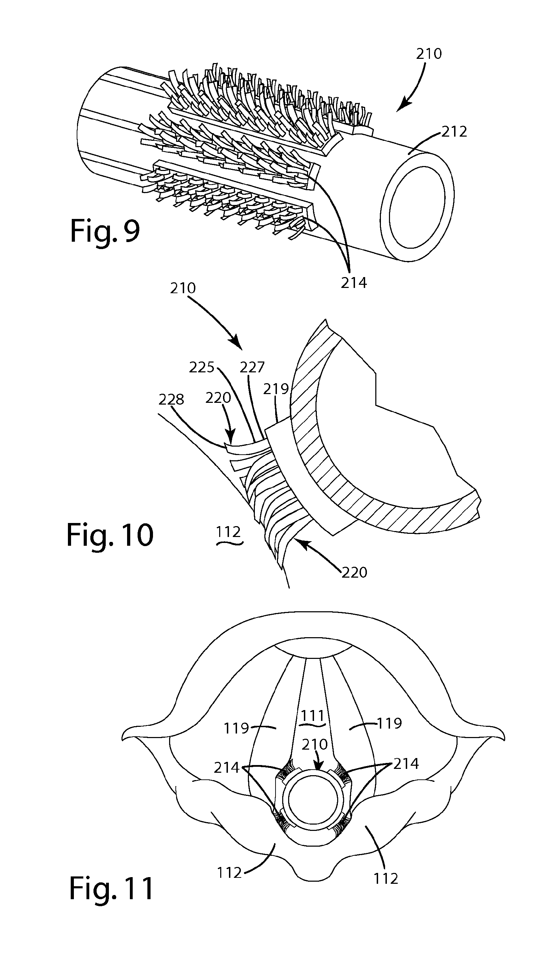

Various other embodiments of the device 10 are contemplated. For example, a second alternative embodiment of the device is illustrated in Figs. 8-11 and generally designated 210. Generally, Figs. 9 and 10 illustrate portions of the device 210, and in particular, portions of the cannula 212 including sensors 214. This embodiment is similar to the above embodiment with a few exceptions. For example, the sensors 214 are in the form a reconfigurable sensor which includes moveable electrodes. More particularly, each individual moveable electrode 220 is attached with a base 219, which is joined with the cannula 212. The moveable electrode can be a flexible electrode, that is, it can bend or otherwise be reconfigure as shown in Fig. 10 when engaged by an anatomical feature, such as a laryngeal muscle 112. With this flexing, the electrodes can be brought within monitoring proximity of the target muscle or nerve in an atraumatic manner. Each of the individual electrodes 220 can include a distal end 228 and a proximal end 227, between which a medial portion 225 is disposed. The proximal end 227 can be joined with a base 219, which again can be joined with a cannula 212. Each electrode can be coated with a desired polymer and can include a metal or otherwise electrically conductive core. A variety of carbon structures also may be suitable for this particular flexible electrodes.

Fig. 11 illustrates the device 210 of the second alternative embodiment with the cannula 210 inserted within the laryngeal space 111. The sensors 214 generally conform to the anatomical geometry of the laryngeal space 111, and in particular, the laryngeal target muscles 112. Upon making contact with the respective laryngeal muscles, the moveable electrodes 214 can reconfigure from a generally straightened mode to a flexed mode (as shown in Fig. 10), where the end of the flexible electrodes 220 move relative to the cannula 212. The flexible electrode thereby reconfigures in shape so as to fit within the laryngeal space yet still adequately contact the target laryngeal muscles 112.

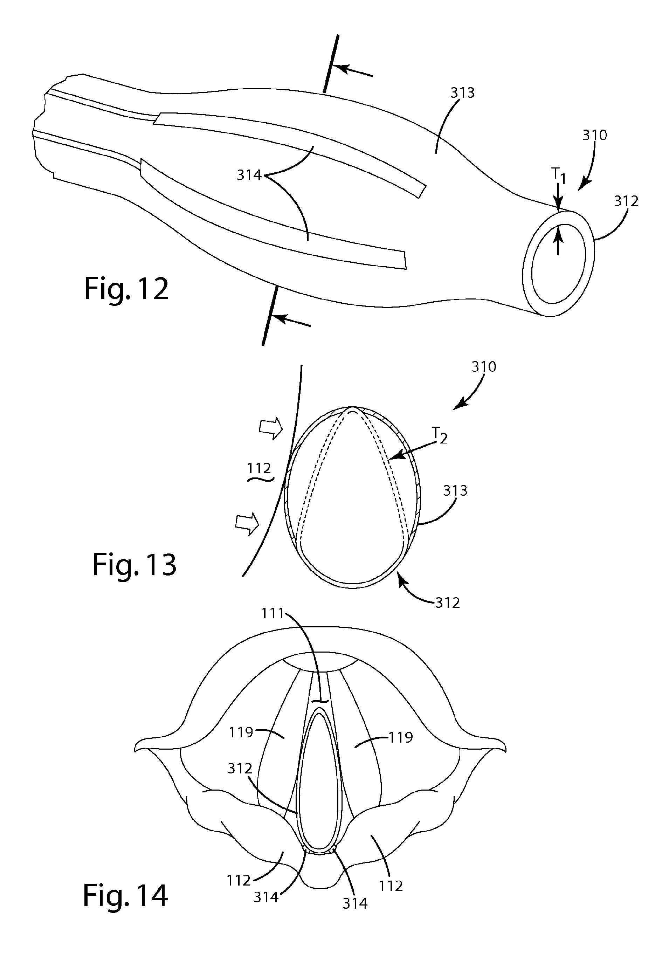

A third alternative embodiment of the device is illustrated in Figs. 12-14 and generally designated 310. Generally, Figs. 12 and 13 illustrate portions of the device 310, and in particular, portions of the cannula 312 including sensors 314. This embodiment is similar to the above embodiment with a few exceptions. For example, the cannula 312 can include a reconfigurable portion 313. Portion 313 is reconfigurable from a first configuration to a second configuration that conforms to the anatomical geometry of at least a portion of a body space, for example, the laryngeal space. As another example, the reconfigurable portion, or the cannula generally, can include a structure that enables it to reconfigure in size, shape or orientation, and in so doing conforms better to the anatomical geometry of the space in which it is placed.

As shown in Fig. 12, the electrodes 314 can be adapted to withstand some flexure due to the reconfiguration of the reconfigurable portion 313. The reconfigurable portion 313 of the cannula 312 can be constructed so as to change in at least one of size and shape so that it can fit through the glottic opening and/or generally conform to the laryngeal space and enhance contact between the sensors 314 and the target laryngeal muscle to measure activity of that muscle. As shown in Figs. 13 and 14, the exterior surface of the reconfigurable portion 313 of the cannula 312 can change in shape when that portion is forced against an object, such as laryngeal muscle 112 and/or 119. Accordingly, the exterior surface of the endotracheal tube conform to the anatomical geometry of the same in an traumatic manner.

To provide this reconfigurability, the reconfigurable portion 313 can include a wall that is of a thickness that is less than the remainder of the cannula. For example, the thickness T₂ shown in Fig. 13 of the reconfigurable portion 313 can be less than that of the thickness T₁ of the wall of the remainder of the cannula 312. As shown in Fig. 12, the difference in thickness can vary, for example, thickness T₂ can be b, ¾, a, ½, ¼ of the thickness T₁. Alternatively, or in combination, the reconfigurable portion 313 of the cannula can be constructed from a different material from the remainder of the cannula 312. As an example, the reconfigurable portion 313 can be constructed from an elastomeric or flexible material having a higher elasticity so that it readily changes in shape and/or forces, such as those exerted on the cannula 312 when inserted through the glottic opening. Fig. 14 illustrates the reconfigurable portion 313 which, due to its increased flexibility relative to the remainder of the cannula, changes in shape and conforms to the anatomical geometry of at least a portion of the glottic opening in an atraumatic manner. In this configuration, the sensors 314 also are brought into monitoring proximity to the target laryngeal muscles 112. With the configuration of the cannula in this embodiment, the contact between the target muscles and the sensors can be enhanced.

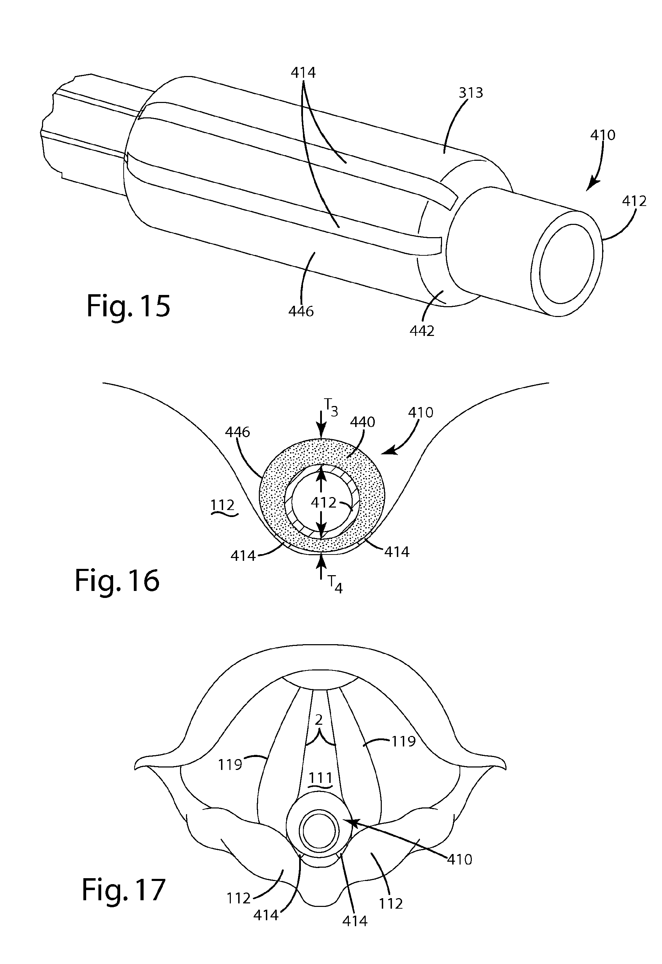

A fourth alternative embodiment of the device is illustrated in Figs. 15-17 and generally designated 410. Generally, Figs. 15-16 illustrate portions of the device 410, and in particular, portions of the cannula 412 including sensors 414. This embodiment is similar to the above embodiments with a few exceptions. For example, the portion of the cannula 412 as illustrated includes a support element 440. This support element 440 is generally joined with the cannula 412 and generally surrounds the cannula 412. The support element 440 includes an outer surface 446 that is adapted to engage the anatomical geometry of the laryngeal space or other body space in which the cannula is positioned. As can be seen, the support element is generally of a size and dimension that is greater than that of the cannula 412. The dimensions of the outer diameter, can be selected based on the amount of space within which the device is to be positioned.

The support element can be integral with the endotracheal tube or a completely separate element that is positioned over the endotracheal tube or cannula 412. Further optionally, the support element can simply be an integral part of the endotracheal tube or cannula. Regardless of the alternative constructions in the embodiments described herein, the endotracheal tube is considered to "include" the support element where the support element is part of the device.

The device 410 can include sensors 414 joined with the support element 440. The sensors 414 can be embedded within the support element 440 or simply attached to an outer surface. The support element can enable movement of the sensors 414 relative to the cannula 412 so that the sensors are within monitoring proximity to a target laryngeal muscle. Accordingly, the activity of the related target nerve can be measured when the nerve is stimulated by an electrical probe such as that described above.

As shown in Fig. 16, the support element 440 can be compressible so that it can reconfigure from a thickness T₃ to T₄ when forced against an object, such as a laryngeal muscle 112. In so doing, with the reduction of thickness, the support element compresses in the area where the force is applied. Generally, with the compressibility of the support element 440, the contact between the sensors 414 and the target laryngeal muscles 112 can be improved.

The support element 440 can be constructed from a variety of materials. As an example, those materials may include compressible materials, such as elastomeric materials, foam materials, closed cell foam materials, an air filled bladder, or combinations of the foregoing.

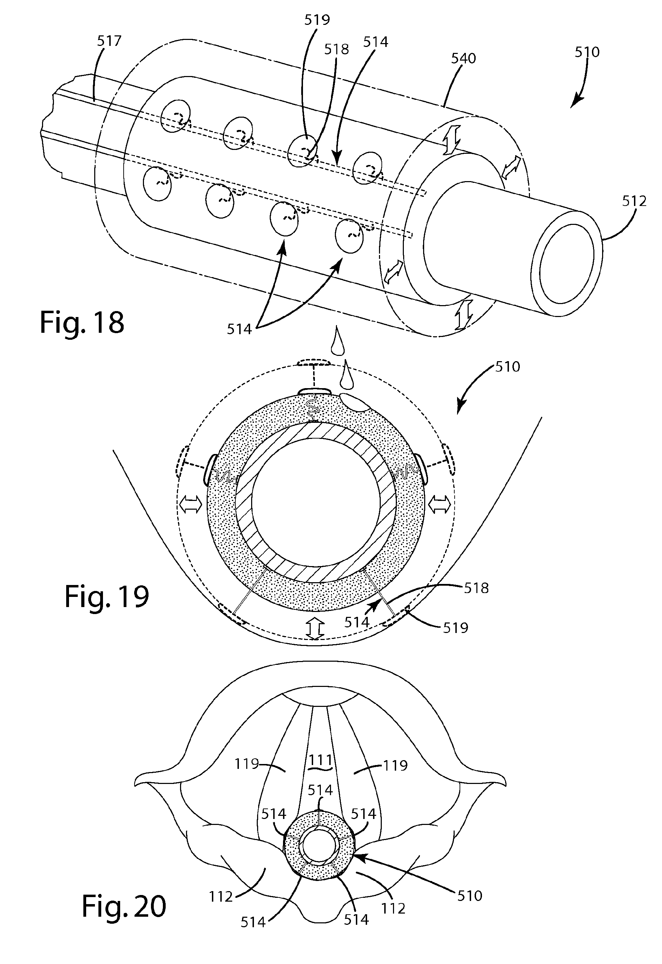

A fifth alternative embodiment of the device is illustrated in Figs. 18-20 and generally designated 510. Generally, Figs. 18-19 illustrate portions of the device 510, and in particular, portions of the cannula 512 including sensors 514. As shown in Fig. 18, the cannula 512 includes another support element 540, which may either be integral with the cannula 512 or separately joined with the cannula. This support element can be adapted to expand or contact, depending on different environmental conditions. As one example, the support element 540 can be constructed from a sponge-like material, which when dry is in a compressed mode. When wetted, the sponge material can expand outward as shown in Figs. 18-19 in broken lines to achieve a greater size and dimension. This greater size and dimension can enhance the contact between the sensors 514 and the target muscles for monitoring of nerves associated with those muscles. Alternatively, the support element 540 can be constructed so that it compresses from an expanded mode to a compressed mode when subjected to liquids. Within this construction, as the support element 540 and cannula 512 enter a body space, secretions of the body space can cause the support element to reduce in size and generally conform to the anatomical geometry of the body space.

In general, when the support element 540 expands, it increases in size and/or dimension within the general body space, for example, the laryngeal space. This in turn can enable the cannula/endotracheal tube to conform to the anatomical geometry of the body space in an atraumatic manner. Similarly where the support element is a compressible element, it can decrease in size when forced toward a target laryngeal muscle. In turn, the endotracheal tube within which the compressible element is included can conform to the anatomical geometry of the body space in an atraumatic manner.

As shown in Figs. 18 and 19, the sensors 514 can be of a particular construction to accommodate the expansion and/or compression of the support element 540, and the relative movement of the outer surface of the support element relative to the cannula 512. For example, the electrodes can include a primary wire 517 that couples to multiple secondary wires 518. These secondary wires 518 can be in a furled or coiled or otherwise accordion-like configuration when the support element is in a compressed mode. Generally, the wires can be considered to be unextended in this configuration. When the support element transitions from a compressed mode to an expanded mode, for example, when it is wetted, the secondary wires 518 unfurl or uncoil as shown in Fig. 19.

The secondary wire can include a portion that is adjacent the outer surface of the support element 540. This portion can provide the desired monitoring of the target muscle. Alternatively, the ends of the secondary wire 518 can be joined with caps 519 that are positioned on the outer surface of the support element. These caps can provide increased surface area for engagement of the sensor 514 with the target nerve, and generally can enhance the engagement of the sensor with a target muscle to ensure that the activity of the nerve is measured when stimulated.
The above description is that of the current embodiment of the invention.

## Claims

1. A nerve or muscle monitoring device comprising:
a cannula (12) configured for insertion into an internal body space of a subject;
a sensor (14) joined with the cannula (12) at a predetermined location, the predetermined location corresponding to at least one of a target muscle and a target nerve when the cannula (12) is positioned in the internal body space of the subject; and
an output element (40) in communication with the sensor (14) the output element (40) providing output indicative of measured activity of at least one of the nerve and muscle when a probe is positioned adjacent or on the target nerve;
wherein the sensor (14) is configured to conform to the anatomical geometry of at least a portion of the internal body space of the subject in an atraumatic manner so that the sensor can measure the activity of the at least one of the target muscle and the target nerve when placed in electrical proximity to the at least one of the target muscle and target nerve, whereby a health care provider is provided with information concerning the location of the target nerve;
wherein the sensor (14) is a multi-sensor array, the sensors (14) being positioned around a majority of the cannula (12); wherein the sensors (14) are combined in a single sheet sensor subdivided into separate detection zones;
**characterised in that** the sensors include electrodes (14) having at least one portion that is configured to move relative to the cannula to which the sensor is joined in order to allow the portion to conform to the anatomical geometry of the space within which it is placed.

2. A nerve monitoring device according to claim 1, wherein the nerve is the laryngeal nerve, the cannula (12) is an endotracheal tube configured for insertion into a laryngeal space of a subject; the target muscle is the laryngeal muscle and the target nerve is the laryngeal nerve, the internal body space is the laryngeal body space.

3. The device of claim 2, wherein at least one of the sensor (14) and the endotracheal tube (12) are reconfigurable from a first configuration to a different second configuration, wherein the second configuration conforms to the anatomical geometry of at least a portion of the laryngeal space.

4. The device of any preceding claim, wherein the sensor includes a flexible electrode having an end projecting away from the cannula, wherein the end is moveable relative to the cannula (12).

5. The device of claim 2, wherein the sensor (14) includes a flexible electrode having an end projecting away from the endotracheal tube, wherein the end (228) is moveable relative to the endotracheal tube so that the flexible electrode can reconfigure in shape to fit within the laryngeal space yet still contact the target laryngeal muscles.

6. The device of any preceding claim, wherein the sensor is electrically conductive and changes in at least one of shape, size, and orientation relative to the cannula so that it is urged into contact with at least one of the target muscle and the target nerve to measure the activity of at least one of the target muscle and the target nerve.

7. The device of any preceding claim comprising an alignment element (16) joined with the cannula and in a fixed orientation relative to the sensor, the alignment element including an alignment indicator (18, 20, 22) which provides output to a health care provider as to the location of the sensor relative to the at least one of target muscle and target nerve, whereby the output can assist the health care provider in reducing the risk of impairment or damage to the target nerve.

8. The device of claim 7, wherein the output is in the form of at least one of an audible alarm, a visual alarm, and movement, whereby a user is informed by the output.

9. The device of any preceding claim, wherein the sensor (14) includes a plurality of electrodes in an array circumferentially disposed around an outer circumference of the cannula, the array configured so that at least two of the electrodes can be in electrical contact with the at least one of the target muscle and target nerve regardless of the rotational direction of the cannula (12) within the internal body space.

10. The device of any preceding claim, wherein the cannula (12) includes a support element (440) adapted to move, preferably relative to the cannula, the support element including an outer surface (446) adapted to engage the anatomical geometry of at least a portion of the internal body space within which the cannula is positioned, the sensors (14) joined with the support element (40), the sensor including a portion or an end mounted on or adjacent the outer surface so that the sensor can measure the activity of the at least one of the target muscle and the target nerve when the sensors (14) is placed in electrical proximity to the at least one of the target muscle and target nerve.

11. The device of claim 10, wherein the support element is at least one of an expanding element that increased in size in the internal body space to enhance engagement with the target muscle, and a compressible element that decreases in size when forced toward the target muscle, preferably wherein the sensor includes a cap positioned on the outer surface of the expanding element.

12. The device of any preceding claim, wherein the cannula (12) is configured to change in shape so that the exterior surface of the cannula conforms to the anatomical geometry of at least a portion of the internal body space of the subject matter in an atraumatic manner.

13. The device of any preceding claim, wherein the cannula includes a portion having at least one wall that is of a different thickness from the thickness of the other portions of the cannula, wherein the wall flexes to conform to the anatomical geometry of at least a portion of the internal body space of the subject in an atraumatic manner, and/or wherein the cannula includes a portion that is more flexible than the remainder of the cannula so that the portion can change in shape and conform to the anatomical geometry of at least a portion of the internal body space of the subject in an atraumatic manner.

14. The device of any preceding claim, wherein the cannula includes a support element that moves the sensor relative to the cannula so that the sensor engages the at least one of the target muscle and the target nerve to measure the activity of the at least one of the target muscle and the target nerve.

## Patentansprüche

1. Nerven- oder Muskelüberwachungseinheit umfassend:
eine Kanüle (12), gestaltet zum Einführen in einen Körperinnenraum eines Subjekts;
einen Sensor (14), der an einem vorbestimmten Ort mit der Kanüle (12) verbunden ist, wobei der vorbestimmte Ort wenigstens einem von einem Zielmuskel und einem Zielnerv entspricht, wenn die Kanüle (12) in dem Körperinnenraum des Subjekts positioniert ist; und
ein Ausgabeelement (40) in Kommunikation mit dem Sensor (14), wobei das Ausgabeelement (40) eine Ausgabe liefert, die für die gemessene Aktivität von wenigstens einem von dem Nerv und dem Muskel indikativ ist, wenn eine Sonde nahe dem oder auf dem Zielnerv positioniert ist;
wobei der Sensor (14) dafür gestaltet ist, der anatomischen Geometrie wenigstens eines Abschnitts des Körperinnenraums des Subjekts auf atraumatische Weise zu entsprechen, so dass der Sensor die Aktivität des wenigstens einen von dem Zielmuskel und dem Zielnerv messen kann, wenn er in elektrischer Nähe zu dem wenigstens einen von dem Zielmuskel und dem Zielnerv angeordnet ist, wodurch ein Gesundheitsfürsorger mit Informationen über den Ort des Zielnervs versorgt wird;
wobei der Sensor (14) ein Multisensorfeld ist, wobei die Sensoren (14) um einen Großteil der Kanüle (12) positioniert sind; wobei die Sensoren (14) in einen einzelnen Blattsensor kombiniert sind, der in getrennte Nachweiszonen aufgeteilt ist;
**dadurch gekennzeichnet, dass** die Sensoren Elektroden (14) enthalten, die wenigstens einen Abschnitt aufweisen, der dafür gestaltet ist, sich relativ zu der Kanüle zu bewegen, mit der der Sensor verbunden ist, um zu erlauben, dass der Abschnitt der anatomischen Geometrie des Raums, innerhalb dessen er angeordnet ist, entspricht.

2. Nervenüberwachungseinheit gemäß Anspruch 1, wobei der Nerv der Kehlkopfnerv ist, die Kanüle (12) ein Endotrachealtubus ist, der zum Einführen in einen Kehlkopfraum eines Subjekts gestaltet ist; der Zielmuskel der Kehlkopfmuskel ist und der Zielnerv der Kehlkopfnerv ist, der Körperinnenraum der Kehlkopfkörperraum ist.

3. Einheit gemäß Anspruch 2, wobei wenigstens eines von dem Sensor (14) und dem Endotrachealtubus (12) von einer ersten Konfiguration zu einer unterschiedlichen, zweiten Konfiguration rekonfigurierbar ist, wobei die zweite Konfiguration der anatomischen Geometrie wenigstens eines Abschnitts des Kehlkopfraums entspricht.

4. Einheit gemäß einem der vorstehenden Ansprüche, wobei der Sensor eine flexible Elektrode mit einem Ende aufweist, das von der Kanüle weg ragt, wobei das Ende relativ zu der Kanüle (12) beweglich ist.

5. Einheit gemäß Anspruch 2, wobei der Sensor (14) eine flexible Elektrode mit einem Ende aufweist, das von dem Endotrachealtubus weg ragt, wobei das Ende (228) relativ zu dem Endotrachealtubus beweglich ist, so dass sich die flexible Elektrode in ihrer Form rekonfigurieren kann, um in den Kehlkopfraum zu passen und dennoch mit den Kehlkopf-Zielmuskeln in Kontakt zu stehen.

6. Einheit gemäß einem der vorstehenden Ansprüche, wobei der Sensor elektrisch leitfähig ist und sich in wenigstens einem von Form, Größe und Orientierung relativ zu der Kanüle verändert, so dass er in Kontakt mit wenigstens einem von dem Zielmuskel und dem Zielnerv gedrückt wird, um die Aktivität von wenigstens einem von dem Zielmuskel und dem Zielnerv zu messen.

7. Einheit gemäß einem der vorstehenden Ansprüche, umfassend ein Ausrichtelement (16), das mit der Kanüle verbunden ist und sich in einer unveränderlichen Orientierung relativ zu dem Sensor befindet, wobei das Ausrichtelement einen Ausrichtungsindikator (18, 20, 22) enthält, der eine Ausgabe über den Ort des Sensors relativ zu dem wenigstens einen von dem Zielmuskel und dem Zielnerv an einen Gesundheitsfürsorger liefert, wodurch die Ausgabe den Gesundheitsfürsorger bei der Verringerung des Risikos einer Beeinträchtigung oder Schädigung des Zielnervs unterstützen kann.

8. Einheit gemäß Anspruch 7, wobei die Ausgabe in der Form von wenigstens einem von einem hörbaren Alarm, einem sichtbaren Alarm und einer Bewegung vorliegt, wodurch ein Anwender über die Ausgabe informiert wird.

9. Einheit gemäß einem der vorstehenden Ansprüche, wobei der Sensor (14) eine Vielzahl von Elektroden in einem Feld umfasst, das rings um einen Außenumfang der Kanüle angeordnet ist, wobei das Feld so gestaltet ist, dass unabhängig von der Rotationsorientierung der Kanüle (12) innerhalb des Körperinnenraums wenigstens zwei der Elektroden in elektrischem Kontakt mit dem wenigstens einen von dem Zielmuskel und dem Zielnerv stehen können.

10. Einheit gemäß einem der vorstehenden Ansprüche, wobei die Kanüle (12) ein Trägerelement (440) enthält, das dafür ausgelegt ist, sich zu bewegen, vorzugsweise relativ zu der Kanüle, wobei das Trägerelement eine Außenoberfläche (446) aufweist, die dafür ausgelegt ist, in die anatomische Geometrie von wenigstens einem Abschnitt des Körperinnenraums, in dem die Kanüle positioniert ist, einzupassen, wobei die Sensoren (14) mit dem Trägerelement (40) verbunden sind, wobei der Sensor einen Abschnitt oder ein Ende aufweist, der/das auf oder nahe der Außenoberfläche befestigt ist, so dass der Sensor die Aktivität von dem wenigstens einen von dem Zielmuskel und dem Zielnerv messen kann, wenn der Sensor (14) in elektrischer Nähe von dem wenigstens einen von dem Zielmuskel und dem Zielnerv angeordnet ist.

11. Einheit gemäß Anspruch 10, wobei das Trägerelement wenigstens eines von einem expandierenden Element, das in dem Körperinnenraum an Größe zunimmt, um das Einpassen an den Zielmuskel zu verbessern, und ein komprimierbares Element ist, das an Größe abnimmt, wenn es zu dem Zielmuskel gedrückt wird, vorzugsweise wobei der Sensor eine Kappe aufweist, die auf der Außenoberfläche des expandierenden Elements angeordnet ist.

12. Einheit gemäß einem der vorstehenden Ansprüche, wobei die Kanüle (12) dafür gestaltet ist, ihre Form zu verändern, so dass die Außenoberfläche der Kanüle der anatomischen Geometrie von wenigstens einem Abschnitt des Körperinnenraums des Subjektmaterials auf atraumatische Weise entspricht.

13. Einheit gemäß einem der vorstehenden Ansprüche, wobei die Kanüle einen Abschnitt aufweist, der wenigstens eine Wand aufweist, die eine andere Dicke als die Dicke der anderen Abschnitte der Kanüle aufweist, wobei sich die Wand verbiegt, um der anatomischen Geometrie von wenigstens einem Abschnitt des Körperinnenraums des Subjekts auf atraumatische Weise zu entsprechen, und/oder wobei die Kanüle einen Abschnitt aufweist, der flexibler als der Rest der Kanüle ist, so dass der Abschnitt seine Form ändern und der anatomischen Geometrie von wenigstens einem Abschnitt des Körperinnenraums des Subjekts auf atraumatische Weise entsprechen kann.

14. Einheit gemäß einem der vorstehenden Ansprüche, wobei die Kanüle ein Trägerelement aufweist, das den Sensor relativ zu der Kanüle bewegt, so dass sich der Sensor an das wenigstens eine von dem Zielmuskel und den Zielnerv anpasst, um die Aktivität des wenigstens einen von dem Zielmuskel und dem Zielnerv zu messen.

## Revendications

1. Dispositif de surveillance d'un muscle ou d'un nerf comprenant :
une canule (12) configurée pour une insertion dans un espace interne du corps d'un sujet ;
un capteur (14) joint à la canule (12) à un emplacement prédéterminé, l'emplacement prédéterminé correspondant à au moins un muscle cible et un nerf cible lorsque la canule (12) est positionnée dans l'espace interne du corps ; et
un élément de sortie (40) en communication avec le capteur (14), l'élément de sortie (40) fournissant une sortie indicative d'activité mesurée d'au moins un du nerf et du muscle lorsqu'une sonde est positionnée de manière adjacente à ou sur le nerf cible ;
où le capteur (14) est configuré pour s'adapter à la géométrie anatomique d'au moins une partie de l'espace interne du corps du sujet de manière atraumatique, afin que le capteur puisse mesurer l'activité de l'au moins du muscle cible et du nerf cible lorsqu'il est placé à proximité électrique de l'au moins un du muscle cible et du nerf cible, moyennant quoi un prestataire de soins de santé se voit transmettre des informations concernant l'emplacement du nerf cible ;
où le capteur (14) est un réseau à capteurs multiples, les capteurs (14) étant positionnés autour d'une majeure partie de la canule (12) ; où les capteurs (14) sont combinés en un capteur à feuille unique subdivisé en zones de détection séparées ;
**caractérisé en ce que** les capteurs comprennent des électrodes (14) ayant au moins une partie qui est configurée pour se déplacer par rapport à la canule à laquelle le capteur est joint afin de permettre à la partie de s'adapter à la géométrie anatomique de l'espace à l'intérieur duquel il est placé.

2. Dispositif de surveillance d'un nerf selon la revendication 1, dans lequel le nerf est le nerf laryngé, la canule (12) est un tube trachéal configuré pour une insertion dans un espace laryngé d'un sujet ; le muscle cible est le muscle laryngé et le nerf cible est le nerf laryngé, l'espace interne du corps est l'espace corporel laryngé.

3. Dispositif selon la revendication 2, dans lequel au moins un du capteur (14) et du tube trachéal (12) est reconfigurable à partir d'une première configuration en une seconde configuration différente, où la seconde configuration s'adapte à la géométrie anatomique d'au moins une partie de l'espace laryngé.

4. Dispositif selon l'une quelconque des revendications précédentes, dans lequel le capteur comprend une électrode flexible ayant une extrémité en saillie s'éloignant de la canule, où l'extrémité est mobile par rapport à la canule (12).

5. Dispositif selon la revendication 2, dans lequel le capteur (14) comprend une électrode flexible ayant une extrémité en saillie s'éloignant du tube trachéal, où l'extrémité (228) est mobile par rapport au tube trachéal de manière à ce que l'électrode flexible puisse reconfigurer sa forme pour s'ajuster à l'intérieur de l'espace laryngé tout en étant en contact avec les muscles laryngés cibles.

6. Dispositif selon l'une quelconque des revendications précédentes, dans lequel le capteur est conducteur électriquement et change, au moins en forme, en taille, et en orientation par rapport à la canule de manière à ce qu'il soit poussé à entrer en contact avec au moins un du muscle cible et du nerf cible pour mesurer l'activité d'au moins du muscle cible et du nerf cible.

7. Dispositif selon l'une quelconque des revendications précédentes comprenant un élément d'alignement (16) joint à la canule et dans une orientation fixe par rapport au capteur, l'élément d'alignement comprenant un indicateur d'alignement (18, 20, 22) qui fournit une sortie d'informations à un prestataire de soins de santé concernant l'emplacement du capteur par rapport à l'au moins un du muscle cible et du nerf cible, moyennant quoi la sortie peut aider le prestataire de soins de santé à réduire le risque de blessure ou de dommage au nerf cible.

8. Dispositif selon la revendication 7, dans lequel la sortie se présente sous la forme d'au moins une indication parmi une alarme audible, une alarme visuelle, et un mouvement, par lequel un utilisateur est informé par la sortie.

9. Dispositif selon l'une quelconque des revendications précédentes, dans lequel le capteur (14) comprend une pluralité d'électrodes dans un réseau disposé de manière circonférentielle, autour d'une circonférence extérieure de la canule, le réseau étant configuré de manière à ce qu'au moins deux des électrodes puissent être en contact électrique avec l'au moins un du muscle cible et du nerf cible indépendamment du sens de rotation de la canule (12) à l'intérieur de l'espace interne du corps.

10. Dispositif selon l'une quelconque des revendications précédentes, dans lequel la canule (12) comprend un élément de support (440) conçu pour se déplacer, de préférence par rapport à la canule, l'élément de support comprenant une surface extérieure (446) conçue pour s'insérer dans la géométrie anatomique d'au moins une partie de l'espace interne du corps, à l'intérieur duquel la canule est positionnée, les capteurs (14) joints à l'élément de support (40), le capteur comprenant une partie ou une extrémité montée sur ou adjacente à la surface extérieure de manière à ce que le capteur puisse mesurer l'activité de l'au moins un du muscle cible et du nerf cible lorsque les capteurs (14) sont placés à une proximité électrique de l'au moins un du muscle cible et du nerf cible.

11. Dispositif selon la revendication 10, dans lequel l'élément de support est au moins un élément d'expansion qui augmente en taille dans l'espace interne du corps pour améliorer l'insertion dans le muscle cible, et un élément compressible qui décroît en taille lorsqu'il est poussé vers le muscle cible, de préférence où le capteur comprend un capuchon positionné sur la surface extérieure de l'élément d'expansion.

12. Dispositif selon l'une quelconque des revendications précédentes, dans lequel la canule (12) est configurée pour changer de forme de manière à ce que la surface extérieure de la canule s'adapter à la géométrie anatomique d'au moins une partie de l'espace interne du corps du sujet traité de manière atraumatique.

13. Dispositif selon l'une quelconque des revendications précédentes, dans lequel la canule comprend une partie ayant au moins une paroi dont l'épaisseur est différente de l'épaisseur des autres parties de la canule, où la paroi fléchit pour s'adapter à la géométrie anatomique d'au moins une partie de l'espace interne du corps du sujet de manière atraumatique, et/ou où la canule comprend une partie qui est plus flexible que le reste de la canule de manière à ce que la partie puisse changer de forme et s'adapter à la géométrie anatomique d'au moins une partie de l'espace interne du corps du sujet de manière atraumatique.

14. Dispositif selon l'une quelconque des revendications précédentes, dans lequel la canule comprend un élément de support qui déplace le capteur par rapport à la canule de manière à ce que le capteur s'insère dans l'au moins un du muscle cible et du nerf cible pour mesurer l'activité de l'au moins un du muscle cible et du nerf cible.
